# EUROPEAN PATENT SPECIFICATION

(11) **EP 3 668 654 B1**
(45) Date of publication and mention of the grant of the patent: **01.05.2024**
(21) Application number: 18756201.2
(22) Date of filing: 14.08.2018
(51) Int. Cl.: A61M 1/02, A61M 1/36, B04B 5/04, B04B 11/02

(54) **FLUID SEPARATION SYSTEM WITH PULSE DAMPENER**
FLÜSSIGKEITSABSCHEIDESYSTEM MIT PULSDÄMPFER
SYSTÈME DE SÉPARATION DE LIQUIDES AVEC AMORTISSEUR D'IMPULSIONS

(30) Priority: 18.08.2017 GB 201713252
(43) Date of publication of application: 24.06.2020
(73) Proprietor: Biosafe S.A., 1262 Eysins (CH)
(72) Inventor: SUBLET, Yannick Andre, 1262 Eysins (CH)
(74) Representative: Bedford, Grant Richard
(86) International application number: PCT/EP2018/072072
(87) International publication number: WO 2019/034671

(56) References cited:
- EP-A1- 0 912 250
- WO-A1-2016/178100
- US-B1- 6 733 433

## Description

### Technical Field of the Invention

The present invention relates to the separation of a biological fluid into its component parts, such as separation of whole blood into red blood cells, white blood cells, platelets and plasma. More specifically the present invention provides a system for use in carrying out such separation.

### Description of Related Art

The components of blood, i.e. red blood cells, white blood cells, platelets and plasma are used for different therapies and therefore a certain amount of donated whole blood is processed in order to separate out these components. In a typical blood donation, around 450 mL of whole blood is collected into a blood collection bag containing an anticoagulant solution. The collected blood is separated into its sub-components by spinning the blood bag for a period of about 10 minutes in a large refrigerated centrifuge. Following centrifugation, each of the components are expressed sequentially from the blood collection bag into separate collection bags.

There has been a desire for more automated, compact and portable systems for collection and separation of biological fluids, that are suitable even for processing relatively small volumes.

US3737096 and US4303193 propose a relatively small centrifugal apparatus attached to collapsible bags. However, these devices have a minimum fixed holding volume which requires a minimum volume usually of about 250 mL to be processed before any components can be collected.

US5316540 discloses a centrifugal processing apparatus corresponding to the where the processing chamber is a flexible processing bag which can be deformed to fill it with biological fluid or empty it by means of a membrane which forms part of the drive unit.

EP0654669-A discloses a centrifugal processing apparatus having two chambers separated by a piston. Before centrifugation, a small quantity of fluid to be processed is taken in *via* an off-centre inlet, and is transferred between the two chambers during centrifugal processing.

EP0912250-B1 teaches a portable and disposable centrifugal apparatus with a processing chamber of variable volume. It can therefore process a variable quantity of biological fluid, even down to very small quantities. US6733433 describes a similar apparatus. Both of these documents, as well as WO2016178100A1, teach control of the movement of the piston by means of a pneumatic system located at the bottom of the chamber that selectively creates either a vacuum or a positive pressure to move the piston up or down as desired. The present inventors recognise a number of problems with a pneumatic system to control movement of the piston, including:
- Low reactivity of piston movement (loop reactivity of 15 seconds)
- Air dilatation/compression effect (also referred to as "stick and slip")
- Limitation in minimal piston speed (no regulation between -100 and +100mbar)
- Sensitive to movement friction of the piston

EP0912250-B1 also suggests use of two peristaltic pumps acting on tubing lines connected to the processing chamber for transferring biological fluid into and out of the chamber. The present inventors recognise that peristaltic pumps may be problematic in that flow jerks are readily generated, which can cause unwanted stress on cells in the biological fluid.

There is therefore scope for a further improved system for processing biological fluids that overcomes these problems.

### Summary of the Invention

Various aspects and embodiments of the present invention are provided, as defined by the appended claims.

The system of the present invention overcomes the problems connected with using compressed air to control movement of the axially moveable member, such that the speed of movement can be more finely controlled and reducing or eliminating problems of stick and slip as well as flow jerk during the movement. The present invention reduces reactivity of movement of the axially moveable member and is very sensitive to piston friction. With known methods it is difficult to control the pressure near to 0 mbar (between -100 and +100 mbar), whereas the present invention allows for smooth movement of the axially moveable member under 1ml/s.

This invention therefore combines the advantages of variable volume with a high quality of regulation of movement of the axially moveable member.

### Brief Description of the Figures

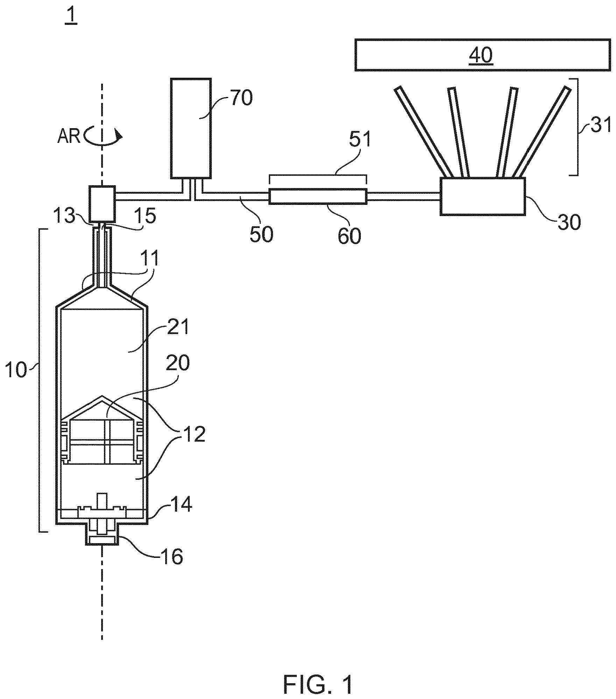

Figure 1 illustrates a non-limiting example of a particular embodiment of the system of the present invention. The various numbered features of Figure 1 can be understood with reference to the description provided herein.

### Detailed Description of the Preferred Embodiments

To more clearly and concisely describe and point out the subject matter of the claimed invention, definitions are provided hereinbelow for specific terms used throughout the present specification and claims. Any exemplification of specific terms herein should be considered as a non-limiting example.

The terms "comprising" or "comprises" have their conventional meaning throughout this application and imply that the agent or composition must have the essential features or components listed, but that others may be present in addition. The term 'comprising' includes as a preferred subset "consisting essentially of".

A "biological fluid" in the context of the present invention is taken to mean any treated or untreated fluid associated with a living organism, including, but not limited to blood, saliva, lymph, cerebrospinal fluid, ascites fluid, and urine. Biological fluid particularly includes blood, including whole blood, warm or cold blood, and stored or fresh blood; treated blood, such as blood diluted with a physiological solution, including but not limited to saline, nutrient, and/or anticoagulant solutions. A liquid cell culture may also be regarded as a biological fluid.

The "components" of a biological fluid will be dependent on the particular biological fluid in question but will include cellular components and the liquid in which they are present. So for example, the components of human blood are red blood cells, white blood cells, platelets and plasma, and the components of a liquid cell culture are the cells being grown and the culture medium.

The term "pulse damper" as used herein is intended to be a generic term to refer to a device that intercepts pressure pulses and/or prevents their creation. A pulse damper is positioned generally adjacent to the source of the flow or pressure disturbance, which is typically a modulating element like a pump or a flow control valve. A pulse damper works by collecting the peak flow, and delivering it back to the system when the rate of mass transfer decreases, thus smoothing and averaging the flow. In the system of the present invention the pulse damper is positioned between the chamber and the pump to reduce flow jerks, which can be a problem when flow into the separation chambers is in waves.

A "centrifugal separating chamber" in the context of the present invention is a hollow centrifuge processing chamber rotatable about an axis of rotation by engagement of the processing chamber with a rotary drive unit. Typically, a centrifugal separating chamber is generally cylindrical in shape such that components are separated concentrically. The separating chamber in one embodiment comprises a generally cylindrical wall extending from an end wall of the processing chamber, this generally cylindrical wall defining therein a hollow open cylindrical space coaxial with the axis of rotation, the axial opening being provided in said end wall coaxial with the generally cylindrical wall to open into the hollow processing chamber. The processing chamber contains within the generally cylindrical wall the axially movable member. Typical materials from which a separating chamber can be made include polypropylene (PP), polycarbonate (PC), polyoxymethylene (POM) and liquid silicone rubber (LSR). In one embodiment the walls of the chamber are made from PP, the axially moveable member is made of PC and the seals are made from LSR. Separating chambers are known in the art and commercially available, e.g. from GE Healthcare's Biosafe.

The "walls" of the centrifugal separating chamber define the outer shape of the chamber as well as the hollow interior. The "hollow interior" is the entire internal space defined by the walls of the chamber.

The "axial opening" positioned at the top end of the centrifugal separating chamber is an opening through which biological fluid to be processed enters the chamber and processed components of said biological fluid exit the chamber. It is of a diameter suitable for the biological fluid to readily pass through without causing damage to any of its components. Said axial opening is typically of narrower diameter than the centrifugal separating chamber. This opening leads into a separation space of variable volume wherein the entire centrifugal processing of the biological fluid takes place.

The "axially movable member" is configured to fluidly isolate the separation space from the remainder of the hollow interior of the centrifugal separating chamber. It is moveable to either intake a selected quantity of biological fluid to be processed into the separation space *via* the axial opening or to express processed biological fluid components from the separation space *via* said opening. In one embodiment, said axially moveable member is a piston. The separation space of variable volume is defined in an upper part of the processing chamber by the walls of the chamber and the axially movable member. Axial movement of the movable member varies the volume of the separation space, the movable member being axially movable within the processing chamber to intake a selected quantity of biological fluid to be processed into the separation space *via* the inlet before or during centrifugal processing and to express processed biological fluid components from the separation space *via* the axial opening after centrifugal processing.

The phrase "at least part of a pump" refers to the fact that a portion of tubing can be regarded as part of the pump. For illustration, non-limiting examples include where the tubing may be acted upon by a peristaltic pump, or it could be a disposable diaphragm (or "membrane") pump.

The "means for monitoring" the position of the axially movable member comprises a detector responsive to changes in the position of the axially moveable member connected to control circuitry. In one embodiment the means for monitoring comprises an optical sensor assembly made from a vertical array of light-emitting diodes, e.g. with light emitting in the infrared spectrum to avoid interference from ambient light. The control circuitry comprises software that includes instructions for operation of the detector. In certain embodiments the control circuitry may take the form of software programmed into a desktop computer, a laptop computer or a smartphone.

Each of said "plurality of containers" is a container suitable for the collection and/or storage of a biological fluid or one of its components. In one embodiment each of said containers is a plastic bag of the type well-known in the field of blood collection and storage.

The term "additive solutions" may be taken to encompass any solution useful in blood collection and storage, e.g. anticoagulant solutions, preservatives, buffers.

The term "in fluid communication" takes its ordinary meaning, which is to say that a fluid may readily pass from one component to the other, either directly or by means of a conduit such as a pipe or tube. The term "in selective fluid communication" means that the path between two components can be selectively closed.

The "plurality of valves" is a distribution valve arrangement that establishes selective communication between the separating chamber and the plurality of containers or for placing the processing chamber and each of the containers either in or out of communication.

A "pump" in the context of the present invention is an apparatus for moving fluid by means of a piston, plunger, or set of rotating vanes.

The term "transferring" as applied to use of the system of the present invention refers to the process of moving the biological fluid into the separation space or of moving the separated components out of the separation space. The act of transferring should be carried out in a manner that causes little to no damage to the components of the biological fluid as it is required for therapeutic applications that these components are intact. Movement of the axially moveable member downwardly in the separation chamber creates a vacuum to intake liquid, and its movement upwardly facilitates transfer of the separated components into their respective conainers.

The word "rotating" as it applies to use of the separation chamber means rotation to speeds suitable for separating a biological fluid into its components within the separation chamber. In one embodiment, a "speed suitable for centrifugal separation" of the biological fluid is in the range 4000-8000 rpm, preferably 5000-7000 rpm, more preferably 5500-6500 rpm.

In one embodiment of the system (1), said pulse dampener (70) comprises an air cavity positioned above said tubing (50). This "air cavity" can be understood to be an air space above the fluid wherein the air acts upon the fluid flow to smooth out flow jerks. By way of illustration, non-limiting examples include where the air cavity is separated from the fluid flow in the tubing by a diaphragm, or where the air cavity is part of a drip chamber with fluid in the bottom where the air takes up the changes in pressure. In one embodiment, said pulse dampener (70) is an air spring. In one embodiment, said pulse dampener (70) is a drip chamber.

In one embodiment, said air cavity is separated from the interior of said tubing (50) by a diaphragm.

In another embodiment of the system (1), said pulse dampener (70) comprises a piston backed by a mechanical spring.

In one embodiment of the system (1), said separating chamber (10) is substantially cylindrical. In one embodiment, the walls (11) of said separating chamber (10) are formed from rigid plastic. In one embodiment, said rigid plastic is polypropylene.

In one embodiment of the system (1), said axially moveable member (20) is formed from rigid plastic. In one embodiment, said rigid plastic is polycarbonate. In one embodiment, said axially moveable member (20) further comprises one or more seals positioned around its outer circumference. In one embodiment, these seals are O-rings. In one embodiment, the seals are formed from a low-friction material, for example silicone or rubber.

In one embodiment, the system (1) further comprises a plurality of containers (40) fluidly connected to said plurality of tubes (31) whereby said chamber (10) and said plurality of containers (40) are in selective fluid communication. Said plurality of containers (40) in one embodiment comprises a container into which biological fluid has been collected. In another embodiment, said plurality of containers (40) comprises one or more containers, each for the collection of one of separated components of said biological fluid. In a further embodiment, said plurality of containers (40) comprises one or more containers containing additive solutions. Additive solutions suitable for the different components of blood, for example, are known to those of skill in the art. In a yet further embodiment, each of said plurality of containers (40) is a blood collection bag of the type widely used in the art, i.e. typically made from a biological fluid-compatible flexible plastic.

In one embodiment, the system (1) further comprises a pump (60) positioned at or around said tubing (50) between said pulse dampener (70) and said valve arrangement (30). In one embodiment, said pump is external to said tubing (50). In one embodiment, said pump (60) is a positive displacement pump. In one embodiment, said pump (60) is a peristaltic pump. In another embodiment, said pump (60) is a membrane pump.

In one embodiment of the system (1) said plurality of valves is independently a stopcock or a pinch valve. In one embodiment, each of said plurality of valves is a stopcock. In one embodiment, each of said plurality of valves is a pinch valve.

In one embodiment, the system (1) further comprises means (81) for controlling operation of the system (1). Any device onto which software can be uploaded for use can constitute such a means. In one embodiment, said means (81) comprises means (82) for monitoring the position of the axially movable member (20), to control the amount of biological fluid taken into said chamber and the expression of separated components.

In one embodiment, the system can be used for processing blood. In one embodiment, said blood is umbilical cord blood. The components of blood that are separated by use of the system when the biological fluid is blood comprise plasma, stem cells and red blood cells.

In one embodiment, the system can be used for processing biological fluids other than blood that contain one or more cell populations of interest, one non-limiting example would be a liquid cell culture.

The system of the present invention is operated generally in the same manner as described for the system of US 6733433. Therefore, for example, to process umbilical cord blood is collected into a bag containing an anticoagulant. The bag containing the collected blood is aseptically connected to axial opening of the centrifugal separating chamber. The valve in the flow path between the bag and the separation space is adjusted to open a path between the bag and the separation space, and the pump is activated to promote movement of the fluid from the bag to the separation space as well as movement of the axially moveable member towards the bottom end of the separating chamber. Centrifugation may start and be stabilised at a speed of around 4000 rpm before the blood enters the separation space. The centrifuge speed may be gradually increased to reach around 6000 rpm, which is maintained for about 5-8 min, after which the centrifuge speed is slowly decreased. Collection of the separated components can take place before the centrifuge has stopped. For collection, the valve between the umbilical cord blood collection bag and the separation space is closed and the valves between the separation space and bags for the separated components are sequentially opened at the same time as activating the pump in the opposite direction to promote movement out of the separation chamber and movement of the axially moveable member towards the top end of the separating chamber. The separated components are collected starting with plasma, followed by platelets contained in the buffy-coat layer, then stem cells, white blood cells and then red blood cells.

The different components can be identified by light absorbance in an optical line sensor, so that at a certain absorbances defined by the software the correct valves are opened to permit the components to be directed to designated bags. Centrifugation is generally stopped following collection of the stem cells and white blood cells to allow a smooth extraction of the remaining red cells into their bag. Optionally, an additive solution can be added to one or more of the bags containing the separated components by opening the appropriate valves to establish flow between a bag containing said additive solution and the selected bag. An example of an additive solution is a preservative solution based on 10 or 20 vol % DMSO, which can contain also a physiological buffer such as phosphate buffer.

Another alternative to isolate the stem cell rich fraction from the buffy-coat is by using density gradient products such as those available under the names Ficoll^{™} and Percoll^{™}. The density gradient product is first introduced into the separation space, followed by introduction of the cord blood, components of the blood are separated into its respective container and its collection is completed when the density gradient appears. Possibly the density gradient product may be introduced during processing. Using Ficoll^{™} would for example consist of first introducing the density gradient into the processing chamber, followed then by blood. After complete introduction of blood into the chamber, a sedimentation period of a few minutes is started. Stem cells and platelets form an interface in front of the gradient, whereas erythrocytes and granulocytes pass through the Ficoll^{™} and are held against the walls of the separation chamber. The piston is then lifted gently as in the standard procedure, the stem cell fraction being collected at the apparition of the first platelets. The effluent line clears up again when Ficoll^{™} exits the chamber.

This written description uses examples to disclose the invention, including the preferred mode, and also to enable any person skilled in the art to practice the invention, including making and using any devices or systems and performing any incorporated methods. The patentable scope of the invention is defined by the claims.

Such other examples are intended to be within the scope of the claims if they have structural elements that do not differ from the literal language of the claims, or if they include equivalent structural elements with insubstantial differences from the literal languages of the claims.

## Claims

1. A system (1) for the separation of a biological fluid into its components, wherein the system (1) comprises:
a centrifugal separating chamber (10) rotatable about an axis of rotation (AR) and having walls (11) that define a hollow interior (12), a top end (13), a bottom end (14) and an axial opening (15) positioned at said top end (13);
an axially movable member (20) contained within said chamber (10) which, along with said walls (11), defines a separation space (21) of variable volume, within said chamber (10);
a plurality of valves (30) wherein each valve is in selective fluid connection with one of a plurality of tubes (31);
at least one container (40) fluidly connected to said plurality of tubes (31) whereby said chamber (10) and said at least one container (40) are in selective fluid communication;
a length of tubing (50) for establishing fluid communication between said chamber (10) and said plurality of valves (30), said tubing including a portion (51) which forms at least a part of a pump (60) for fluid pumping within the tubing (50); and
a pulse dampener (70) positioned along said tubing (50) between said chamber (10) and the position for said portion (51); **characterized in that**:
a valve of the plurality of valves (30) in the flow path between a container of said at least one container (40) and the separation space (21) is adjustable to open a path between the container and the separation space (21), and wherein the pump (60) is activatable to promote movement of the biological fluid from the container to the separation space (21) as well as movement of the axially movable member (20) towards the bottom end (14) of the separating chamber (10).

2. The system (1) as defined in Claim 1, comprising a plurality of containers (40) and wherein the valve between the container and the separation space (21) is closable and further valves of the plurality of valves (30) between the separation space (21) and other containers (40) of the plurality of containers (40) are sequentially openable for collecting separated components of the biological fluid whilst the pump (60) is activated to promote movement of the biological fluid out of the separation chamber (10) and movement of the axially movable member (20) towards the top end (13) of the separating chamber (10).

3. The system (1) as defined in Claim 1 or Claim 2, further comprising a pulse dampener (70) positioned along the tubing (50) between the separating chamber (10) and the position for the portion (51).

4. The system (1) as defined in Claim3 wherein the pump (60) is positioned at or around said tubing (50) between said pulse dampener (70) and said valve arrangement (30).

5. The system (1) as defined in any one of Claims 1-4, wherein the separating chamber (10) is substantially cylindrical.

6. The system (1) as defined in any one of Claims 1-5, wherein at least one of said at least one container (40) comprises a blood collection bag.

7. The system (1) as defined in any one of Claims 1-6, wherein said pump is external to said tubing (50), and is optionally a positive displacement pump, a peristaltic pump or a membrane pump.

8. The system (1) as defined in any one of Claims 1-7 further comprising means (81) for controlling operation of the system (1), and wherein optionally said means (81) comprises further means (82) for monitoring a position of the axially movable member (20).

9. The system (1) as defined in any one of Claims 1-8 wherein the walls (11) of said separating chamber (10) are formed from rigid plastic, and optionally wherein said rigid plastic comprises polypropylene.

10. The system (1) as defined in any one of Claims 1-9 wherein said axially movable member (20) is formed from rigid plastic, and optionally wherein said rigid plastic comprises polycarbonate.

11. The system (1) as defined in any one of Claims 1-10 wherein said axially movable member (20) further comprises one or more seals positioned around its outer circumference.

12. The system (1) as defined in Claim 11 wherein said seals comprise O-rings, a low-friction material, silicone and/or rubber.

13. A method for the separation of a biological fluid into its components using the system (1) as defined in any one of Claims 1-12, wherein said method comprises:
transferring a biological fluid from a container (40) into which it has been collected into a separation space (21) by moving an axially movable member (20) towards a bottom end (14) of a separating chamber (10);
rotating the separation chamber (10) at a speed suitable for centrifugal separation of the biological fluid within the separation space (21) to obtain a number of separated components of the biological fluid;
transferring each separated component from the separation space (21) into one of a plurality of containers (40) designated for collection of the separated component by selective opening of a plurality of valves (30) and movement of the axially movable member (20) towards a top end (13) of the separating chamber (10); and
**characterised in that** the movement of the axially movable member (20) is achieved by creation of a pressure differential by a pump (60) for fluid pumping within the tubing (50) positioned at or around said tubing (50) between said pulse dampener (70) and said valve arrangement (30).

14. The method as defined in Claim 13, wherein the step of transferring a biological fluid from a container (40) into the separation space (21) comprises transferring of blood collected in a blood collection bag into the separation space (21).

15. The method as defined in Claim 13, wherein the step of transferring a biological fluid from a container (40) into the separation space (21) comprises transferring a liquid cell culture into the separation space (21).

## Patentansprüche

1. System (1) zur Trennung eines biologischen Fluids in ihre Bestandteile, wobei das System (1) umfasst:
eine Zentrifugaltrennkammer (10), welche um eine Rotationsachse (AR) drehbar ist und Wände (11) aufweist, welche einen hohlen Innenraum (12), ein oberes Ende (13), ein unteres Ende (14) und eine axiale Öffnung (15) definieren, welche an dem oberen Ende (13) positioniert ist;
ein axial bewegliches Element (20), welches in der Kammer (10) enthalten ist, welches zusammen mit den Wänden (11) einen Trennraum (21) mit variablem Volumen innerhalb der Kammer (10) definiert;
eine Vielzahl von Ventilen (30), wobei jedes Ventil in selektiver Fluidverbindung mit einem einer Vielzahl von Rohren (31) steht;
zumindest einen Behälter (40), welcher mit der Vielzahl von Rohren (31) fluidisch verbunden ist, wobei die Kammer (10) und der zumindest eine Behälter (40) in selektiver Fluidkommunikation stehen;
eine Rohrleitungslänge (50) zum Herstellen einer Fluidkommunikation zwischen der Kammer (10) und der Vielzahl von Ventilen (30), wobei die Rohrleitung einen Abschnitt (51) beinhaltet, welcher zumindest einen Teil einer Pumpe (60) zum Pumpen von Fluid in der Rohrleitung (50) bildet; und
einen Impulsdämpfer (70), welcher entlang der Rohrleitung (50) zwischen der Kammer (10) und der Position für den Abschnitt (51) positioniert ist; **dadurch gekennzeichnet, dass**:
ein Ventil der Vielzahl von Ventilen (30) in dem Strömungsweg zwischen einem Behälter des zumindest einen Behälters (40) und dem Trennraum (21) einstellbar ist, um einen Weg zwischen dem Behälter und dem Trennraum (21) zu öffnen, und wobei die Pumpe (60) aktivierbar ist, um eine Bewegung des biologischen Fluids von dem Behälter zu dem Trennraum (21) sowie eine Bewegung des axial beweglichen Elements (20) zu dem unteren Ende (14) der Trennkammer zu fördern (10).

2. System (1) nach Anspruch 1, umfassend eine Vielzahl von Behältern (40), und wobei das Ventil zwischen dem Behälter und dem Trennraum (21) verschließbar ist und weitere Ventile der Vielzahl von Ventilen (30) zwischen dem Trennraum (21) und anderen Behältern (40) der Vielzahl von Behältern (40) nacheinander öffenbar sind, um getrennte Bestandteile des biologischen Fluids zu sammeln, während die Pumpe (60) aktiviert ist, um eine Bewegung des biologischen Fluids aus der Trennkammer (10) und eine Bewegung des axial beweglichen Elements (20) zu dem oberen Ende (13) der Trennkammer (10) zu fördern.

3. System (1) nach Anspruch 1 oder Anspruch 2, weiter einen Impulsdämpfer (70) umfassend, welcher entlang der Rohrleitung (50) zwischen der Trennkammer (10) und der Position für den Abschnitt (51) positioniert ist.

4. System (1) nach Anspruch 3, wobei die Pumpe (60) an der oder um die Rohrleitung (50) herum zwischen dem Impulsdämpfer (70) und der Ventilanordnung (30) positioniert ist.

5. System (1) nach einem der Ansprüche 1-4, wobei die Trennkammer (10) im Wesentlichen zylindrisch ist.

6. System (1) nach einem der Ansprüche 1-5, wobei zumindest einer des zumindest einen Behälters (40) einen Blutsammelbeutel umfasst.

7. System (1) nach einem der Ansprüche 1-6, wobei sich die Pumpe außerhalb der Rohrleitung (50) befindet und optional eine Verdrängerpumpe, eine peristaltische Pumpe oder eine Membranpumpe ist.

8. System (1) nach einem der Ansprüche 1-7, weiter Mittel (81) zum Steuern des Betriebs des Systems (1) umfassend, und wobei die Mittel (81) optional weitere Mittel (82) zum Überwachen einer Position des axial beweglichen Elements (20) umfassen.

9. System (1) nach einem der Ansprüche 1-8, wobei die Wände (11) der Trennkammer (10) aus starrem Kunststoff gebildet sind und wobei der starre Kunststoff optional Polypropylen umfasst.

10. System (1) nach einem der Ansprüche 1-9, wobei das axial bewegliche Element (20) aus starrem Kunststoff gebildet ist und wobei optional der starre Kunststoff Polycarbonat umfasst.

11. System (1) nach einem der Ansprüche 1-10, wobei das axial bewegliche Element (20) weiter eine oder mehrere Dichtungen umfasst, welche um seinen Außenumfang herum positioniert sind.

12. System (1) nach Anspruch 11, wobei die Dichtungen O-Ringe, ein reibungsarmes Material, Silikon und/oder Gummi umfassen.

13. Verfahren zur Trennung eines biologischen Fluids in ihre Bestandteile unter Verwendung des Systems (1) nach einem der Ansprüche 1-12, wobei das Verfahren umfasst:
Übertragen eines biologischen Fluids aus einem Behälter (40), in welchem sie gesammelt wurde, in einen Trennraum (21) durch Bewegen eines axial beweglichen Elements (20) zu einem unteren Ende (14) einer Trennkammer (10);
Drehen der Trennkammer (10) mit einer Geschwindigkeit, welche zur zentrifugalen Trennung des biologischen Fluids innerhalb des Trennraums (21) geeignet ist, um eine Anzahl getrennter Bestandteile des biologischen Fluids zu erhalten;
Übertragen jedes getrennten Bestandteils aus dem Trennraum (21) in einen einer Vielzahl von Behältern (40), welche zum Sammeln des getrennten Bestandteils bestimmt sind, durch selektives Öffnen einer Vielzahl von Ventilen (30) und eine Bewegung des axial beweglichen Elements (20) zu einem oberen Ende (13) der Trennkammer (10); und
**dadurch gekennzeichnet, dass** die Bewegung des axial beweglichen Elements (20) durch Erzeugung einer Druckdifferenz durch eine Pumpe (60) zum Pumpen von Fluid innerhalb der Rohrleitung (50) erreicht wird, welche an der oder um die Rohrleitung (50) herum zwischen dem Impulsdämpfer (70) und der Ventilanordnung (30) positioniert ist.

14. Verfahren nach Anspruch 13, wobei der Schritt des Übertragens eines biologischen Fluids aus einem Behälter (40) in den Trennraum (21) das Übertragen von in einem Blutsammelbeutel gesammeltem Blut in den Trennraum (21) umfasst.

15. Verfahren nach Anspruch 13, wobei der Schritt des Übertragens eines biologischen Fluids aus einem Behälter (40) in den Trennraum (21) das Übertragen einer flüssigen Zellkultur in den Trennraum (21) umfasst.

## Revendications

1. Système (1) pour la séparation d'un fluide biologique en ses composants, dans lequel le système (1) comprend :
une chambre de séparation centrifuge (10) pouvant tourner autour d'un axe de rotation (AR) et comportant des parois (11) qui définissent un intérieur creux (12), une extrémité supérieure (13), une extrémité inférieure (14) et une ouverture axiale (15) positionnée au niveau de ladite extrémité supérieure (13) ;
un élément pouvant se déplacer axialement (20) contenu à l'intérieur de ladite chambre (10) qui, conjointement avec lesdites parois (11), définit un espace de séparation (21) de volume variable, à l'intérieur de ladite chambre (10) ;
une pluralité de vannes (30), dans lequel chaque vanne est en liaison fluidique sélective avec un tube d'une pluralité de tubes (31) ;
au moins un récipient (40) relié fluidiquement à ladite pluralité de tubes (31), selon lequel ladite chambre (10) et ledit au moins un récipient (40) soient en communication fluidique sélective ;
une longueur de tube (50) pour établir une communication fluidique entre ladite chambre (10) et ladite pluralité de vannes (30), ledit tube incluant une partie (51) qui forme au moins une partie d'une pompe (60) pour un pompage de fluide à l'intérieur du tube (50) ; et
un amortisseur d'impulsions (70) positionné le long dudit tube (50) entre ladite chambre (10) et la position de ladite partie (51) ; **caractérisé en ce que** :
une vanne de la pluralité de vannes (30) dans le trajet d'écoulement entre un récipient dudit au moins un récipient (40) et l'espace de séparation (21) est réglable pour ouvrir un chemin entre le récipient et l'espace de séparation (21), et dans lequel la pompe (60) peut être activée pour favoriser un déplacement du fluide biologique depuis le récipient jusqu'à l'espace de séparation (21) ainsi qu'un déplacement de l'élément pouvant se déplacer axialement (20) vers l'extrémité inférieure (14) de la chambre de séparation (10).

2. Système (1) selon la revendication 1, comprenant une pluralité de récipients (40) et dans lequel la vanne entre le récipient et l'espace de séparation (21) peut être fermée et d'autres vannes de la pluralité de vannes (30) entre l'espace de séparation (21) et d'autres récipients (40) de la pluralité de récipients (40) peuvent être ouvertes de manière séquentielle pour collecter des composants séparés du fluide biologique pendant que la pompe (60) est activée pour favoriser un déplacement du fluide biologique hors de la chambre de séparation (10) et un déplacement de l'élément pouvant se déplacer axialement (20) vers l'extrémité supérieure (13) de la chambre de séparation (10).

3. Système (1) selon la revendication 1 ou la revendication 2, comprenant en outre un amortisseur d'impulsions (70) positionné le long du tube (50) entre la chambre de séparation (10) et la position de la partie (51).

4. Système (1) selon la revendication 3, dans lequel la pompe (60) est positionnée au niveau, ou autour, dudit tube (50) entre ledit amortisseur d'impulsions (70) et ledit agencement de vanne (30).

5. Système (1) selon l'une quelconque des revendications 1-4, dans lequel la chambre de séparation (10) est sensiblement cylindrique.

6. Système (1) selon l'une quelconque des revendications 1-5, dans lequel au moins un dudit au moins un récipient (40) comprend une poche de collecte de sang.

7. Système (1) selon l'une quelconque des revendications 1-6, dans lequel ladite pompe est externe au dit tube (50) et est facultativement une pompe volumétrique, une pompe péristaltique ou une pompe à membrane.

8. Système (1) selon l'une quelconque des revendications 1-7, comprenant en outre des moyens (81) pour commander le fonctionnement du système (1) et dans lequel, facultativement, lesdits moyens (81) comprennent des moyens supplémentaires (82) pour surveiller une position de l'élément pouvant se déplacer axialement (20).

9. Système (1) selon l'une quelconque des revendications 1-8, dans lequel les parois (11) de ladite chambre de séparation (10) sont formées à partir de plastique rigide et, facultativement, dans lequel ledit plastique rigide comprend du polypropylène.

10. Système (1) selon l'une quelconque des revendications 1-9, dans lequel ledit élément pouvant se déplacer axialement (20) est formé à partir de plastique rigide, et, facultativement, dans lequel ledit plastique rigide comprend du polycarbonate.

11. Système (1) selon l'une quelconque des revendications 1-10, dans lequel ledit élément pouvant se déplacer axialement (20) comprend en outre un ou plusieurs joints d'étanchéité positionnés autour de sa circonférence externe.

12. Système (1) selon la revendication 11, dans lequel lesdits joints d'étanchéité comprennent des joints toriques, un matériau à faible friction, de la silicone et/ou du caoutchouc.

13. Procédé pour la séparation d'un fluide biologique en ses composants à l'aide du système (1) selon l'une quelconque des revendications 1-12, dans lequel ledit procédé comprend :
le transfert d'un fluide biologique depuis un récipient (40) dans lequel il a été collecté dans un espace de séparation (21) en déplaçant un élément pouvant se déplacer axialement (20) vers une extrémité inférieure (14) d'une chambre de séparation (10) ;
la rotation de la chambre de séparation (10) à une vitesse appropriée pour une séparation centrifuge du fluide biologique à l'intérieur de l'espace de séparation (21) pour obtenir un certain nombre de composants séparés du fluide biologique ;
le transfert de chaque composant séparé de l'espace de séparation (21) dans un récipient d'une pluralité de récipients (40) désignés pour la collecte du composant séparé par l'ouverture sélective d'une pluralité de vannes (30) et un déplacement de l'élément pouvant se déplacer axialement (20) vers une extrémité supérieure (13) de la chambre de séparation (10) ; et
**caractérisé en ce que** le déplacement de l'élément pouvant se déplacer axialement (20) est obtenu par la création d'un différentiel de pression par une pompe (60) pour un pompage de fluide à l'intérieur du tube (50) positionné au niveau, ou autour, dudit tube (50) entre ledit amortisseur d'impulsions (70) et ledit agencement de vanne (30).

14. Procédé selon la revendication 13, dans lequel l'étape de transfert d'un fluide biologique depuis un récipient (40) dans l'espace de séparation (21) comprend le transfert du sang collecté dans une poche de collecte de sang dans l'espace de séparation (21).

15. Procédé selon la revendication 13, dans lequel l'étape de transfert d'un fluide biologique depuis un récipient (40) dans l'espace de séparation (21) comprend le transfert d'une culture cellulaire liquide dans l'espace de séparation (21).
